(19) Europäisches Patentamt
European Patent Office
Office européen des brevets

(11) **EP 3 801 674 B1**

(12) **EUROPÄISCHE PATENTSCHRIFT**

(45) Veröffentlichungstag und Bekanntmachung des Hinweises auf die Patenterteilung:
**08.05.2024 Patentblatt 2024/19**

(21) Anmeldenummer: **19729720.3**

(22) Anmeldetag: **06.06.2019**

(51) Internationale Patentklassifikation (IPC):
*A61M 60/13* (2021.01)   *A61M 60/178* (2021.01)
*A61M 60/216* (2021.01)   *A61M 60/523* (2021.01)
*A61M 60/816* (2021.01)

(52) Gemeinsame Patentklassifikation (CPC):
**A61M 60/13; A61M 60/178; A61M 60/216;
A61M 60/523; A61M 60/816**

(86) Internationale Anmeldenummer:
**PCT/EP2019/064779**

(87) Internationale Veröffentlichungsnummer:
**WO 2019/234148 (12.12.2019 Gazette 2019/50)**

(54) **IMPLANTIERBARES, VENTRIKULÄRES UNTERSTÜTZUNGSSYSTEM**

IMPLANTABLE VENTRICULAR ASSIST SYSTEM

SYSTÈME DE SUPPORT VENTRICULAIRE IMPLANTABLE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **06.06.2018 DE 102018208913**

(43) Veröffentlichungstag der Anmeldung:
**14.04.2021 Patentblatt 2021/15**

(73) Patentinhaber: **Kardion GmbH
70376 Stuttgart (DE)**

(72) Erfinder: **SCHLEBUSCH, Thomas, Alexander
71272 Renningen (DE)**

(74) Vertreter: **Pfiz, Thomas et al
Pfiz/Gauss Patentanwälte PartmbB
Tübinger Strasse 26
70178 Stuttgart (DE)**

(56) Entgegenhaltungen:
**EP-A1- 2 570 143       WO-A1-2016/066180
DE-A1-102009 007 216**

EP 3 801 674 B1

**Beschreibung**

Die Erfindung betrifft eine Verarbeitungseinheit sowie ein implantierbares, ventrikuläres Unterstützungssystem. Die Erfindung findet insbesondere Anwendung bei (voll-)implantierten Linksherz-Unterstützungssystemen (LVAD).

Implantierte Linksherz-Unterstützungssysteme (LVAD) existieren hauptsächlich in zwei Ausführungsvarianten. Einerseits gibt es (perkutane) minimalinvasive Linksherz-Unterstützungssysteme. Die zweite Variante sind unter der Brustkorböffnung invasiv implantierte Linksherz-Unterstützungssysteme. Die erste Variante fördert Blut direkt aus dem linken Ventrikel in die Aorta, da das (perkutane) minimalinvasive Linksherz-Unterstützungssystem mittig in der Aortenklappe positioniert ist. Die zweite Variante fördert das Blut von apikal aus dem linken Ventrikel über einen Bypass-Schlauch in die Aorta.

Die Aufgabe eines kardialen Unterstützungssystems ist die Förderung von Blut. Hierbei hat das sog. Herz-Zeit-Volumen (HZV, üblicherweise angegeben in Liter pro Minute) eine hohe klinische Relevanz. Das Herz-Zeit-Volumen betrifft hierbei mit anderen Worten den Gesamtvolumenstrom an Blut aus einem Ventrikel, insbesondere vom linken Ventrikel hin zur Aorta. Entsprechend eingängig ist das Bestreben, diesen Parameter als Messwert während des Betriebs eines kardialen Unterstützungssystems zu erheben.

Je nach Unterstützungsgrad, der den Anteil des durch ein Fördermittel wie etwa eine Pumpe des Unterstützungssystems geförderten Volumenstroms zum Gesamtvolumenstrom an Blut vom Ventrikel hin zur Aorta beschreibt, gelangt ein gewisser Volumenstrom über den physiologischen Weg durch die Aortenklappe in die Aorta. Das Herz-Zeit-Volumen bzw. der Gesamtvolumenstrom ($Q_{HZV}$) vom Ventrikel hin zur Aorta ist demnach üblicherweise die Summe aus Pumpenvolumenstrom ($Q_p$) und Aortenklappen-Volumenstrom (Qa).

Ein etabliertes Verfahren zur Bestimmung des Herz-Zeit-Volumens ($Q_{HZV}$) im klinischen Umfeld ist die Verwendung von Dilutionsverfahren, die jedoch alle auf einen transkutan eingeführten Katheter setzen und daher nur während einer Herzoperation Herz-Zeit-Volumen-Messdaten liefern können. Ein etabliertes Verfahren zur Messung des Pumpenvolumenstroms ($Q_p$) ist die Korrelation aus den Betriebsparametern des Unterstützungssystems, vor allem der elektrischen Leistungsaufnahme, eventuell ergänzt um weitere physiologische Parameter wie den Blutdruck. Auch die Integration dedizierter Ultraschall-Messtechnik in ein Unterstützungssystem wurde bereits vorgeschlagen.

Eine (voll-)implantierte Erfassung des Herz-Zeit-Volumens, also von $Q_{HZV}$, insbesondere durch das Unterstützungssystem selbst, war bisher noch nicht vorgeschlagen oder realisiert worden. Voll-implantiert bedeutet hierbei insbesondere, dass die für die Erfassung erforderlichen Mittel sich vollständig im Körper des Patienten befinden und dort verbleiben. Dies ermöglicht es, das Herz-Zeit-Volumen auch außerhalb einer Herzoperation zu erfassen.

Die Impedanzkardiografie ist ein Verfahren zur Bestimmung des sog. Cardiac Output mittels extrakorporaler Impedanzmessungen. Dabei kommen vier Elektroden zum Einsatz. Über zwei Elektroden wird ein kleiner Wechselstrom eingespeist, über zwei weitere Elektroden der resultierende Spannungsabfall gemessen. Da Blut eine höhere Leitfähigkeit als umgebendes Gewebe, vor allem als die luftgefüllte Lunge aufweist, ist die Blutvolumenänderung im Thorax über einen Herzzyklus als Impedanzänderung nachweisbar. Die Impedanzkardiografie wird extrakorporal, üblicherweise durch Ring- oder Klebeelektroden um Hals und Bauch angewendet.

Ebenfalls bekannt ist die Verwendung linksventrikulärer Impedanz zusammen mit dem Elektrokardiogramm zur Erkennung abnormaler mechanischer Kontraktionen des Herzmuskels bei LVAD-Patienten. Hierbei wird ein intrakardiales EKG mit einer linksventrikulären Impedanzmessung kombiniert, um abnormale Kontraktionen des Ventrikels zu detektieren. Die Messung wird über das EKG synchronisiert. Die Messung erfolgt qualitativ, eine Volumenbestimmung findet nicht statt.

Der Einsatz von Impedanzmessungen zur quantitativen Volumenbestimmung ist im Bereich des Harnblasenvolumens bekannt. Dabei werden vier Elektroden auf der Außenseite eines Katheters verwendet, der lediglich zu Messzwecken in die Harnblase eingeführt und anschließend wieder entfernt wird. Es hat sich gezeigt, dass die gemessene Impedanz invers abhängig vom Harnblasenvolumen ist. Ein Problem des Verfahrens ist jedoch die starke Abhängigkeit von der Urinleitfähigkeit.

Für einige weitere Beispiele relevanten Stand der Technik siehe EP2570143A1, WO2016/066180A1, und DE102009007216A1.

Ausgehend hiervon liegt der Erfindung die Aufgabe zugrunde, ein Verfahren zum Betreiben eines implantierten, ventrikulären Unterstützungssystems für die Erfassung besonderer Parameter weiter zu verbessern sowie ein entsprechendes vorteilhaftes System anzugeben.

Zur Lösung dieser Aufgabe wird die in den unabhängigen Patentansprüchen 1 und 8 angegebene Merkmalskombination vorgeschlagen. Vorteilhafte Ausgestaltungen und Weiterbildungen der Erfindung ergeben sich aus den abhängigen Ansprüchen.

Hier vorgeschlagen wird darüber hinaus ein Verfahren (nicht beansprucht) zum Betreiben eines implantierten, ventrikulären Unterstützungssystems, umfassend folgende Schritte:

a) Bestimmen eines ersten Impedanzparameters zu einem ersten Zeitpunkt mittels des Unterstützungssystems,

b) Bestimmen eines zweiten Impedanzparameters zu einem zweiten Zeitpunkt mittels des Unterstützungssystems,

c) Zumindest Ermitteln einer Änderung des Impedanzparameters unter Verwendung des ersten Impedanzparameters und des zweiten Impedanzparameters oder Vergleichen zumindest des ersten oder zweiten Impedanzparameters mit einem Schwellenwert.

Das Unterstützungssystem dient vorzugsweise der Förderung von Fluid. Das ventrikuläre Unterstützungssystem ist bevorzugt ein kardiales Unterstützungssystem. Vorzugsweise dient das Verfahren zur Bestimmung eines Fluid-Gesamtvolumenstroms (durch einen Querschnitt im Bereich des Unterstützungssystems) aus einem Ventrikel eines Herzens, insbesondere von einem (linken) Ventrikel eines Herzens hin zur Aorta im Bereich eines (voll-)implantierten, (links-)ventrikulären (Herz-)Unterstützungssystems, und/oder zur Bestimmung eines Aortenklappen- bzw. Bypassvolumenstroms, der am Unterstützungssystem vorbei strömt. Bei dem Fluid handelt es sich regelmäßig um Blut. Das Unterstützungssystem ist bevorzugt am Ausgang des linken Ventrikels des Herzens bzw. der linken Herzkammer angeordnet. Besonders bevorzugt ist das Unterstützungssystem in Aortenklappenposition angeordnet. Mit dem Gesamtvolumenstrom ist insbesondere der gesamte Volumenstrom durch ein Blutgefäß bzw. durch einen Querschnitt des Blutgefäßes gemeint. Bei dem Blutgefäß handelt es sich beispielsweise um die Aorta, insbesondere bei einem Linksherz-Unterstützungssystem, oder um den gemeinsamen Stamm (Truncus pulmonalis) in die beiden Lungenarterien, insbesondere bei einem Rechtsherz-Unterstützungssystem. Das Verfahren ist insbesondere zur Bestimmung des gesamten Herz-Zeit-Volumens (HZV, Formelzeichen $Q_{HZV}$) eines Patienten, insbesondere mit (voll-)implantiertem linksventrikulären Herzunterstützungssystem (LVAD) in Aortenklappenposition und/oder durch das Unterstützungssystem selbst geeignet.

Das Verfahren basiert insbesondere auf der Integration einer ventrikulären Impedanzmessung, insbesondere Impedanzspektroskopie in ein (links-)ventrikuläres (Herz-)Unterstützungsssystem (LVAD), vorzugsweise zur Bestimmung des Herz-Zeit-Volumens (HZV) und/oder des Aortenklappen- bzw. Bypassvolumenstroms. Darüber hinaus kann das Verfahren zur Bestimmung des Unterstützungsgrads beitragen. Das Verfahren ermöglich in vorteilhafter Weise, dass das Herz-Zeit-Volumen und/oder der Bypassvolumenstrom auch außerhalb des OP-Szenarios mit vergleichbarer Qualität wie bei Verwendung eines Dilutionskatheters zur Verfügung gestellt werden kann. Dies ist von besonderem Vorteil, da das Herz-Zeit-Volumen ($Q_{HZV}$) eine größere klinische Relevanz als der meist verwendete Pumpenvolumenstrom ($Q_p$) hat, der nur den Fluss durch das Unterstützungssystem selbst quantifiziert. Die hier vorgeschlagene Lösung zeichnet sich insbesondere durch die Nutzung von rein im LVAD integrierter Sensorik aus, es ist beispielsweise keine separate Ultraschallmanschette um die Aorta erforderlich.

In Schritt a) erfolgt ein Bestimmen (mindestens) eines ersten Impedanzparameters zu einem ersten Zeitpunkt mittels des Unterstützungssystems. Bevorzugt erfolgt in Schritt a) ein Bestimmen eines ersten Ventrikelvolumens zu einem ersten Zeitpunkt mittels einer Impedanzmessung mittels des Unterstützungssystems bzw. mittels des ersten Impedanzparameters. Dies bedeutet mit anderen Worten insbesondere, dass das Unterstützungssystem selbst den Impedanzparameter bzw. das Ventrikelvolumen bestimmt, insbesondere misst. In Schritt b) erfolgt ein Bestimmen (mindestens) eines zweiten Impedanzparameters zu einem zweiten Zeitpunkt mittels des Unterstützungssystems. Bevorzugt erfolgt in Schritt b) ein Bestimmen eines zweiten Ventrikelvolumens zu einem zweiten Zeitpunkt mittels einer Impedanzmessung mittels des Unterstützungssystems. Dies bedeutet mit andern Worten insbesondere, dass in den Schritten a) und b) ein Bestimmen mindestens eines Impedanzparameters und/oder eines Ventrikelvolumens im zeitlichen Verlauf erfolgt. Der erste und der zweite Zeitpunkt unterscheiden sich voneinander. Vorzugsweise liegt der zweite Zeitpunkt zeitlich nach dem ersten Zeitpunkt. Es können eine Vielzahl von Impedanzparametern bzw. Ventrikelvolumina zu weiteren Zeitpunkten bestimmt werden. Kurze Abstände zwischen den Zeitpunkten sind für eine Differenzbildung zur Ermittlung des Volumenstroms besonders vorteilhaft. Das Ventrikelvolumen ist insbesondere durch die Ventrikelwand bzw. die in der Art eines Beutels geformte Herzkammerwand sowie durch zwei Herzklappen begrenzt, von denen eine auch als Aortenklappe bezeichnet wird. Das Ventrikelvolumen wird im Wesentlichen durch das Fluidvolumen (hier insbesondere Blutvolumen) im Ventrikel bestimmt. Die Unterschiede im Ventrikelvolumen ergeben sich insbesondere aufgrund der Kontraktion des Herzmuskels und tragen üblicherweise zur Förderung von Blut durch den Kreislauf eines Menschen, ggf. Patienten bei.

Bei dem Impedanzparameter kann es sich beispielsweise um eine (Bio-)Impedanz, insbesondere von Fluid und/oder Gewebe im Bereich des Unterstützungssystems, handeln. Als Impedanzparameter können auch Rohdaten eines Analog-Digital-Wandlers (einheitenlose Werte) einer Impedanzmesseinrichtung dienen. Bevorzugt handelt es sich bei dem Impedanzparameter um eine Ventrikelimpedanz. Dies bedeutet mit anderen Worten insbesondere, dass in den Schritten a) und b) jeweils eine Ventrikelimpedanz ermittelt wird. Die Ventrikelimpedanz ist regelmäßig vom Ventrikelvolumen, insbesondere dem Fluidvolumen im Ventrikel und der Fluidleitfähigkeit (hier insbesondere Blutleitfähigkeit) abhängig. Darüber hinaus kann auch die den Ventrikel umgebende Muskulatur einen Impedanzanteil liefern. Der Einfluss der Blutleitfähigkeit kann durch separate Impedanzmessung in bekanntem Probenvolumen bestimmt werden. Hier bietet sich insbesondere eine Messung im definierten Volumen der (Zulauf-)Kanüle an. Die Impedanzmessung dient insbesondere der Bestimmung der pulsatilen Volumenänderung des Ventrikels während der Systole. Die Impedanz-Daten können in einer vorteilhaften Ausgestaltung durch Drucksensordaten plausibilisiert werden.

Gemäß der Erfindung erfolgt die Bestimmung der Impedanz des Ventrikels (unbekannten Volumens) über Elektroden, die an und/oder in dem Unterstützungssystem integriert sind. Bevorzugt sind die Elektroden (zur Ventrikelimpedanzmessung) auf oder in der Oberfläche, insbesondere auf oder in der Außenoberfläche des Unterstützungssystems (umlaufend) angeordnet. Besonders bevorzugt sind die Elektroden auf oder in der Außenoberfläche einer (Zulauf-)Kanüle des Unterstützungssystems (umlaufend) angeordnet.

In Schritt c) erfolgt ein Ermitteln einer (zeitlichen) Änderung des Impedanzparameters unter Verwendung des ersten Impedanzparameters und des zweiten Impedanzparameters. Alternativ oder kumulativ kann in Schritt c) ein Vergleichen zumindest des ersten oder zweiten Impedanzparameters mit einem Schwellenwert erfolgen. Vorzugsweise erfolgt ein Ermitteln einer (zeitlichen) Änderung einer Ventrikelimpedanz unter Verwendung des ersten Impedanzparameters und des zweiten Impedanzparameters. Bei dem Schwellenwert handelt es sich vorzugsweise um einen (vor-)definierten und/oder konstanten Schwellenwert. Der Schwellenwert kann beispielsweise durch eine Kalibrierung (in-vivo) als eine insbesondere untere Schwelle für den Impedanzparamter bzw. entsprechende Rohdatenwerte ermittelt, insbesondere festlegt werden. Die untere Schwelle ist insbesondere so dimensioniert, dass ein Kollabieren des Ventrikels und/oder eine Saugwirkung, die zu einem Festsaugen des Unterstützungssystems an der Ventrikelinnenwand führt, vermieden werden kann. Bevorzugt erfolgt in Schritt c) ein Ermitteln einer (zeitlichen) Änderung des Ventrikelvolumens unter Verwendung des ersten Impedanzparameters und/oder des ersten Ventrikelvolumens und des zweiten Impedanzparameters und/oder des zweiten Ventrikelvolumens. Alternativ oder kumulativ kann in Schritt c) ein Vergleichen des ersten und/oder zweiten Impedanzparameters und/oder Ventrikelvolumens mit einem Mindestventrikelvolumen erfolgen. Das Ermitteln der Änderung des Impedanzparameters bzw. Ventrikelvolumens erfolgt insbesondere, wenn das Unterstützungssystem in einem Patienten mit einer Teilunterstützung (geringer bis hoher Unterstützungsgrad) implantiert ist. Das Vergleichen mit dem Schwellenwert bzw. Mindestventrikelvolumen erfolgt insbesondere, wenn das Unterstützungssystem in einem Patienten mit einer Vollunterstützung (Unterstützungsgrad = 100%) implantiert ist. Wen nur ein Vergleichen mit dem Schwellenwert bzw. Mindestventrikelvolumen erfolgen soll, kann einer der Schritte a) oder b) entfallen. Darüber hinaus können das Ermitteln der Änderung und das Vergleichen auch kumulativ, insbesondere zeitgleich oder zumindest teilweise parallel durchgeführt werden, beispielsweise bei einem genesenden Kurzzeit-(voll-)Unterstützungspatienten. Alternativ oder kumulativ zu dem unteren Schwellenwert bzw. Mindestventrikelvolumen kann ein oberer Schwellenwert bzw. Maximalventrikelvolumen für das Vergleichen vorgesehen sein. Der obere Schwellenwert bzw. das Mindestventrikelvolumen ist insbesondere so dimensioniert, dass eine zu hohe Belastung, insbesondere Dehnung der Ventrikelwand, vermieden werden kann.

Bei Patienten mit einer Teilunterstützung kann insbesondere auch die Änderung des Impedanzparameters bzw. Ventrikelvolumens während der Systole (Anspannungs- bzw. Blut-Ausströmungsphase des Herzens) zur Förderung des Bluts durch den Kreislauf, mit anderen Worten zur Aufrechterhaltung oder Erhöhung des Fluid-Gesamtvolumenstroms aus dem Ventrikel bzw. des Herz-Zeit-Volumens beitragen. Insbesondere bei diesen Patienten kommt dem Ermitteln der Änderung des Impedanzparameters bzw. Ventrikelvolumens besondere Bedeutung zu. Bevorzugt erfolgt eine Bestimmung der pulsatilen Impedanzparameter- bzw. Volumenänderung des Ventrikels während der Systole. Dies bedeutet mit anderen Worten insbesondere, dass in Schritt c) die (pulsatile bzw. muskulär bedingte) Änderung des Impedanzparameters bzw. Ventrikelvolumens bevorzugt während der Systole ermittelt wird. Besonders bevorzugt wird die Differenz zwischen dem Impedanzparameter bzw. Ventrikelvolumen am Systolen-Anfang und dem Impedanzparameter bzw. Ventrikelvolumen am Systolen-Ende gebildet. Hierzu kann beispielhaft der erste Zeitpunkt der Systolen-Anfang und der zweite Zeitpunkt das Systolen-Ende sein. Bei dieser beispielhaften Darstellung sollte jedoch berücksichtigt werden, dass die Abtastrate des Impedanzparameters bzw. Ventrikelvolumens hoch genug sein sollte, um das Nyquist-Theorem für zu erwartende Ventrikelkontraktionsfrequenzen zu erfüllen, beispielsweise 60 1/s.

Bei Patienten mit einer Vollunterstützung (insbesondere bei einem kontinuierlich arbeitenden Unterstützungssystem) spielt die Änderung des Impedanzparameters bzw. Ventrikelvolumens eine insbesondere eher untergeordnete Rolle. Bei diesen Patienten ist vielmehr darauf zu achten, dass der Impedanzparameter bzw. das Ventrikelvolumen nicht einen unteren Schwellenwert bzw. ein Mindestventrikelvolumen unterschreitet. Bei dem Mindestventrikelvolumen handelt es sich vorzugsweise um ein definiertes bzw. vorbestimmtes und/oder konstantes Mindestventrikelvolumen. Das Mindestventrikelvolumen ist insbesondere so dimensioniert, dass ein Kollabieren des Ventrikels und/oder eine Saugwirkung, die zu einem Festsaugen des Unterstützungssystems an der Ventrikelinnenwand führt, vermieden werden kann. Der Vergleich kann in der Verarbeitungseinheit durchgeführt werden. Hierzu kann die Messeinrichtung den (ersten und/oder zweiten) Impedanzparameter bzw. das (erste und/oder zweite) Ventrikelvolumen als Ausgangsgröße der Verarbeitungseinheit bereitstellen. Der Impedanzparamter bzw. das Ventrikelvolumen kann einen Regelparameter für das Unterstützungssystem darstellen, der vorzugswiese oberhalb des unteren Schwellenwertes bzw. des Mindestventrikelvolumens zu halten ist. Darüber hinaus kann eine Impedanzparamter-zu-Schwellenwert- bzw. Ventrikelvolumen-zu-Mindestventrikelvolumen-Differenz einen Regelparameter für das Unterstützungssystem darstellen. Eine Verarbeitungseinheit des Unterstützungssystems kann diesen Regelparameter als Ausgangsgröße, insbesondere einer Steuereinheit des Unterstützungssystems, die vorzugsweise die Leistung eines Elektromotors des Unterstützungssystems und damit insbesondere auch die (Blut-)Förderleistung des Unterstützungssystems regelt, bereitstellen. So kann beispielsweise die Förderleistung des Unterstützungssystems reduziert werden, wenn ein Ansaugen der Ventrikelwand droht. Darüber hinaus kann beispielsweise die Förderleistung des Unterstützungssystems erhöht werden, wenn eine zu hohe Dehnung der Ventrikelwand droht.

Insbesondere wenn in den Schritten a) und b) eine Vielzahl von Impedanzparametern bzw. Ventrikelvolumina bestimmt werden, ist es besonders vorteilhaft, dass mindestens eine Modellkurve an die Impedanzparameter bzw. Ventrikelvolumina angepasst wird. Dann ist es weiterhin bevorzugt, wenn mit den Modellparametern bzw. der mindestens einen Modellkurve weiter gearbeitet wird.

Nach einer vorteilhaften Ausgestaltung wird vorgeschlagen, dass mit dem (jeweiligen) Impedanzparameter ein Ventrikelvolumen bestimmt wird. Dies bedeutet mit anderen Worten insbesondere, dass zum Bestimmen des Ventrikelvolumens eine Impedanzmessung durchgeführt bzw. aus der Impedanzmessung ein Ventrikelvolumen bestimmt wird. Bevorzugt wird mit dem ersten Impedanzparameter ein erstes Ventrikelvolumen und mit dem zweiten Impedanzparameter ein zweites Ventrikelvolumen bestimmt.

Gemäß der Erfindung wird vorgeschlagen, dass die (jeweiligen) Impedanzparameter über mindestens zwei Elektroden gemessen werden, die an dem Unterstützungssystem angeordnet sind. Besonders bevorzugt wird der erste und der zweite Impedanzparameter (die jeweilige Ventrikelimpedanz) über mindestens zwei, vorteilhaft vier Elektroden gemessen. Es kann hierzu ein (kleiner) Patientenhilfsstrom, beispielsweise 50 kHz, 100 µA (Wechselstrom) zur Messung genutzt werden. Weiterhin bevorzugt sind die Elektroden derart voneinander beabstandet, dass deren Erfassungsbereich bzw. Erfassungsvolumen das Ventrikelvolumen umfasst. Der Erfassungsbereich bzw. das Erfassungsvolumen betrifft insbesondere den Bereich bzw. das Volumen, der bzw. das von den Strompfaden zwischen den Elektroden abgedeckt ist. Dies betrifft mit anderen Worten insbesondere den Bereich bzw. das Volumen, durch den bzw. das sich die Strompfade zwischen den Elektroden erstrecken. Bevorzugt umfassen die mindestens zwei Elektroden mindestens zwei Elektrodenpaare, insbesondere jeweils umfassend eine Stromelektrode und eine Spannungsmessungselektrode. Dabei sollten die Elektroden eines Paares dicht beieinander liegen. Die Paare sollten jedoch zueinander beabstandet sein. Vorteilhaft ist eine Anordnung, bei der die Stromelektroden außen und die Spannungsmessungselektroden innen liegen (in der Art einer Vierleitermessung). Der Erfassungsbereich bzw. das Erfassungsvolumen vergrößert sich in der Regel mit zunehmendem Abstand zwischen den Elektrodenpaaren Der Abstand ist vorzugsweise so zu bestimmen, dass möglichst das gesamte Volumen des Ventrikels erfasst werden kann, jedoch umliegendes Gewebe und/oder umliegende Organe, insbesondere die Lunge, möglichst nicht im Erfassungsbereich liegen.

Nach einer weiteren vorteilhaften Ausgestaltung wird vorgeschlagen, dass, insbesondere zum Bestimmen des ersten und/oder zweiten Impedanzparameters, eine Impedanzmessung bei unterschiedlichen (Wechselstrom-)Frequenzen durchgeführt wird. Dies kann in vorteilhafter Weise dazu beitragen, dass der Einfluss umliegenden Gewebes, insbesondere der Herzmuskulatur, auf die Impedanzmessung reduziert werden kann. Besonders bevorzugt wird eine (Bio-)Impedanzspektroskopie durchgeführt. Vorzugsweise werden die Frequenzen so gewählt, dass eine Bestimmung der Hintergrundimpedanz des Herzmuskels, besonders bevorzugt periodisch, erfolgen kann bzw. erfolgt. Diese Hintergrundimpedanz kann bei der Impedanzmessung des Ventrikelvolumens berücksichtigt werden.

Nach einer vorteilhaften Ausgestaltung wird vorgeschlagen, dass die Leitfähigkeit des Fluids durch eine Impedanzmessung in einem definierten Volumen des Unterstützungssystems ermittelt wird. Dies trägt insbesondere dazu bei, dass das Unterstützungssystem die Leitfähigkeit des Fluids, die einen Einfluss auf die Impedanzmessung hat, selbst ermitteln kann. Bevorzugt liegt das definierte Volumen im Innern einer (Zulauf-)Kanüle des Unterstützungssystems. Besonders bevorzugt wird das definierte Volumen von der Innenoberfläche oder einem Teil der Innenoberfläche der Kanüle des Unterstützungssystems begrenzt. Darüber hinaus kann das definierte Volumen (in einer Richtung entlang der Kanüle) von zwei Elektroden begrenzt werden. Vorzugsweise ist zumindest eine der (begrenzenden) Elektroden dabei auf oder in der Innenoberfläche der Kanüle angeordnet. Weiterhin bevorzugt sind mindestens zwei, vorteilhaft vier Elektroden zur Leitfähigkeitsmessung auf oder in der Innenoberfläche der Kanüle (umlaufend) angeordnet.

Nach einer vorteilhaften Ausgestaltung wird vorgeschlagen, dass weiterhin ein Fluid-Volumenstrom ermittelt wird, der durch das Unterstützungssystems strömt. Dies betrifft mit anderen Worten insbesondere einen Fluid-Volumenstrom der nur durch das Unterstützungssystems selbst hindurch fließt. Bei diesem Fluid-Volumenstrom handelt es sich üblicherweise um den sog. Pumpenvolumenstrom ($Q_p$), der nur den Fluss durch das Unterstützungssystem selbst quantifiziert. Ist dieser Wert zusätzlich zu dem Gesamtvolumenstrom bzw. Herz-Zeit-Volumen ($Q_{HZV}$) bekannt, so kann aus dem Verhältnis von $Q_p$ zu $Q_{HZV}$ (d. h. $Q_p/Q_{HZV}$) der sogenannte Unterstützungsgrad berechnet werden. Zur Bestimmung des Pumpenvolumenstroms kann ein Eingangs im Zusammenhang mit dem Stand der Technik erörtertes, etabliertes Verfahren zur Messung des Pumpenvolumenstroms verwendet werden. Beispielsweise kann der Pumpenvolumenstrom auf Basis von Differenzdruck und Motorkennfeld oder durch explizite Doppler-Ultraschallmessung ermittelt werden. Bevorzugt wird der Pumpenvolumenstrom mittels eines Ultraschallsensors ermittelt, der beispielsweise in einer Spitze des Unterstützungssystems untergebracht sein kann. Bevorzugt wird der zeitliche Verlauf des Fluid-Volumenstroms bzw. Pumpenvolumenstroms ermittelt. Dieser kann mit einem zeitlichen Verlauf des Fluid-Gesamtvolumenstroms verglichen werden. Der Unterstützungsgrad kann als Regelparameter für das Unterstützungssystem bereitgestellt werden.

Nach einer vorteilhaften Ausgestaltung wird vorgeschlagen, dass ein Fluid-Gesamtvolumenstrom im Bereich des Unterstützungssystems ermittelt wird. Bevorzugt ist ein Ermitteln des Fluid-Gesamtvolumenstroms unter Verwendung des ersten Ventrikelvolumens und des zweiten Ventrikelvolumens. In Fällen in denen der Fluid-Gesamtvolumenstrom im Wesentlichen der zeitlichen Ableitung des Ventrikelvolumens entspricht, kann eine (näherungsweise) Ermittlung des Fluid-Gesamtvolumenstroms in der Art eines Differenzenquotienten erfolgen, der der Quotient ($\Delta V/\Delta t$) der Ventrikelvolumendifferenz ($\Delta V$ = zweites Ventrikelvolumen - erstes Ventrikelvolumen) zur Zeitdifferenz ($\Delta t$ = zweiter Zeitpunkt (z. B. Systolen-Ende) - erster Zeitpunkt (z. B. Systolen-Anfang) ist. Fälle in denen der Fluid-Gesamtvolumenstrom im Wesentlichen der zeitlichen Ableitung des Ventrikelvolumens entspricht, können insbesondere bei geringen Unterstützungsgraden und/oder pulsatil arbeitenden Unterstützungssystemen, beispielsweise gesteuert durch einen Drucksensor im Ventrikel oder einen EKG-Sensor, auftreten. Darüber hinaus kann auch eine Abschätzung des gesamten Herz-Zeit-Volumens (HZV) über den gemessenen Pumpenvolumenstrom und das gemessene Ventrikelvolumen erfolgen. Bevorzugt wird der Fluid-Gesamtvolumenstrom bzw. das Herz-Zeit-Volumen unter Verwendung einer zeitlichen Abtastung des Ventrikelvolumens und des Fluid-Volumenstroms bzw. Pumpenvolumenstroms gebildet. Der Fluid-Gesamtvolumenstrom bzw. das Herz-Zeit-Volumen ($Q_{HZV}$) kann in der Regel (jedenfalls bei kontinuierlich arbeitenden Unterstützungssystemen) aus folgendem Zusammenhang zwischen zeitlicher Änderung des Ventrikelvolumens ($\delta V/\delta t$) und dem Basis-Pumpenvolumenstrom ($Q_{P,Diastole}$) ermittelt werden:

$$Q_{HZV}(t) = Q_{P,Diastole} - \frac{\partial V}{\partial t}$$

Bevorzugt wird der ermittelte Fluid-Gesamtvolumenstrom als Regelparameter für das Unterstützungssystem bereitgestellt. Eine Verarbeitungseinheit des Unterstützungssystems kann diesen Regelparameter als Ausgangsgröße, insbesondere einer Steuereinheit des Unterstützungssystems, die vorzugsweise die Leistung eines Elektromotors des Unterstützungssystems und damit insbesondere auch die (Blut-)Förderleistung des Unterstützungssystems regelt, bereitstellen.

Weiterhin bevorzugt wird ein Aortenklappen- bzw. Bypassvolumenstrom ($Q_A$) ermittelt. Besonders bevorzugt wird mittels der Ventrikelvolumenänderung und des Pumpenvolumenstroms die Bypassströmung (Aortenklappen- bzw. Bypassvolumenstrom) am Unterstützungssystem vorbei durch die Aortenklappe quantifiziert:

$$Q_A(t) = -\frac{\partial V}{\partial t} + Q_{P,Diastole} - Q_P(t)$$

Der Bypassvolumenstrom kann als Regelparameter für das Unterstützungssystem bereitgestellt werden.

Nach einem weiteren Aspekt wird eine Verarbeitungseinheit vorgeschlagen, eingerichtet zur Durchführung eines hier vorgeschlagenen Verfahrens. Die Verarbeitungseinheit kann einen Mikroprozessor umfassen, der auf einen Speicher zugreifen kann. Die Verarbeitungseinheit empfängt vorzugsweise Daten von der Messeinrichtung.

Gemäß der Erfindung wird ein implantierbares, ventrikuläres Unterstützungssystem nach Anspruch 1 vorgeschlagen.

Bei dem Unterstützungssystem handelt es sich vorzugsweise um ein linksventrikuläres Herzunterstützungssystem (LVAD) bzw. ein perkutanes, minimalinvasives Linksherz-Unterstützungssystem. Weiterhin bevorzugt ist dieses voll-implantierbar. Das bedeutet mit anderen Worten insbesondere, dass die zur Erfassung erforderlichen Mittel, insbesondere ein Impedanz-Sensor und/oder Pumpenvolumenstromsensor sich vollständig im Körper des Patienten befinden und dort verbleiben. Besonders bevorzugt ist das Unterstützungssystem so eingerichtet bzw. dazu geeignet, dass es zumindest teilweise in einem Ventrikel, bevorzugt dem linken Ventrikel eines Herzens und/oder einer Aorta, insbesondere in Aortenklappenposition angeordnet werden kann.

Weiterhin bevorzugt umfasst das Unterstützungssystem eine Kanüle, insbesondere Zulaufkanüle, eine Strömungsmaschine, wie etwa eine Pumpe und/oder einen Elektromotor. Der Elektromotor ist dabei regelmäßig ein Bestandteil der Strömungsmaschine. Die (Zulauf-)Kanüle ist vorzugsweise so eingerichtet, dass sie im implantierten Zustand Fluid aus einem (linken) Ventrikel eines Herzens hin zu der Strömungsmaschine führen kann. Das Unterstützungssystem ist vorzugsweise länglich und/oder schlauchartig gebildet. Bevorzugt sind die Zulaufkanüle und die Strömungsmaschine im Bereich einander gegenüberliegender Enden des Unterstützungssystems angeordnet.

Gemäß der Erfindung wird vorgeschlagen, dass die Messeinrichtung mindestens zwei Elektroden umfasst, über die eine Impedanz gemessen werden kann. Bevorzugt sind mindestens vier Elektroden für die Messung einer Ventrikelimpedanz vorgesehen. Besonders bevorzugt sind die Elektroden derart eingerichtet und angeordnet, dass die Impedanz des (gesamten) Ventrikels bestimmt werden kann.

Die im Zusammenhang mit dem Verfahren erörterten Details, Merkmale und vorteilhaften Ausgestaltungen können entsprechend auch bei der hier vorgestellten Verarbeitungseinheit und/oder dem Unterstützungssystem auftreten und umgekehrt. Insoweit wird auf die dortigen Ausführungen zur näheren Charakterisierung der Merkmale vollumfänglich Bezug genommen.

Die hier vorgestellte Lösung sowie deren technisches Umfeld werden nachfolgend anhand der Figuren näher erläutert. Es ist darauf hinzuweisen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und/oder Erkenntnissen aus anderen Figuren und/oder der vorliegenden Beschreibung zu kombinieren. Es zeigen schematisch:

Fig. 1     ein implantierbares Unterstützungssystem,

Fig. 2     ein implantiertes, ventrikuläres Unterstützungssystem in einem Herz,

Fig. 3     das Unterstützungssystem aus Fig. 2,

Fig. 4     ein implantiertes, ventrikuläres Unterstützungssystem, und

Fig. 5     ein schematisches Ablaufdiagramm des beschriebenen Verfahrens.

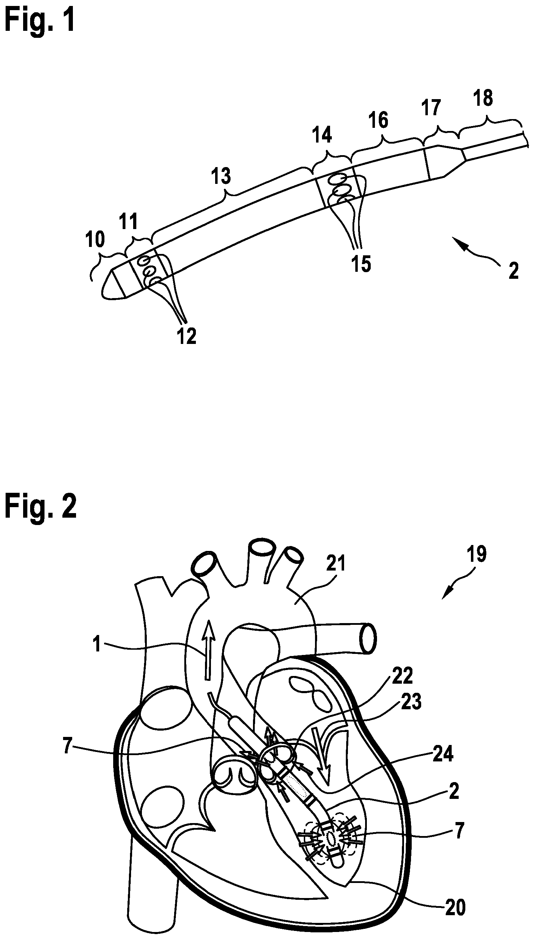

Fig. 1 zeigt schematisch ein implantierbares Unterstützungssystem 2. Das Unterstützungssystem 2 ist hier beispielshaft ein linksventrikuläres Unterstützungssystem (LVAD). Das Unterstützungssystem 2 umfasst eine Spitze 10, die Sensorik enthalten kann, einen Zulaufkäfig 11 mit Zulauföffnungen 12 zur Ansaugung von Fluid (hier: Blut), eine biegsame Kanüle 13, einen Laufradkäfig 14 mit Turbinenrad (hier nicht dargestellt) und Austrittsöffnungen 15 für das Blut, einen Elektromotor 16, ein hinteres Ende 17 (sog. Backend), das Sensorik enthalten kann, und ein Anschlusskabel 18.

Fig. 2 zeigt schematisch ein implantiertes, ventrikuläres Unterstützungssystem 2 in einem Herz 19. Das Unterstützungssystem 2 unterstützt das Herz 19, indem es dazu beiträgt Blut aus dem (linken) Ventrikel 20 in die Aorta 21 zu fördern. Das Unterstützungssystem 2 wird hierzu in der Aortenklappe 22 verankert, wie Fig. 2 verdeutlicht. Bei einem Unterstützungsgrad von 100% fördert das Unterstützungssystem 2 (LVAD) den kompletten Blutvolumenstrom. Der Unterstützungsgrad beschreibt den Anteil des durch ein Fördermittel wie etwa eine Pumpe des Unterstützungssystems 2 bzw. durch das

Unterstützungssystem 2 hindurch geförderten Volumenstroms zum Gesamtvolumenstrom an Blut vom Ventrikel 20 hin zur Aorta 21.

Bei einem Unterstützungsgrad von 100% sind demnach der Fluid-Gesamtvolumenstrom 1 aus dem Ventrikel 20, der Herzklappenvolumenstrom 23 in den Ventrikel 20 sowie der Fluid-Volumenstrom 7 durch das Unterstützungssystem 2 identisch. Der Aortenklappen- bzw. Bypass-Volumenstrom 24 (Formelzeichen: $Q_a$) ist folglich dabei Null. Der Fluid-Gesamtvolumenstrom 1 kann auch als (gesamtes) Herz-Zeit-Volumen (HZV, Formelzeichen: $Q_{HZV}$) beschrieben werden. Der Fluid-Volumenstrom 7 kann auch als sog. Pumpenvolumenstrom (Formelzeichen: $Q_p$) bezeichnet werden der nur den Fluss durch das Unterstützungssystem selbst quantifiziert. Der Unterstützungsgrad kann somit aus dem Verhältnis $Q_p/Q_{HZV}$ berechnet werden.

Bei geringeren Unterstützungsgraden und gesünderen Herzen mit starker Ventrikelkontraktion erfüllt das Herz 19 seine Funktion weiterhin zu einem gewissen Anteil, sodass während der Systole (Herzmuskel zieht sich zusammen und verdrängt durch die Volumenabnahme des Ventrikels 20 Blut in die Aorta 21) ein pulsatiler Volumenstromanteil 24 (Bypass) durch die Herz- bzw. Aortenklappe 22 entsteht. Gleichzeitig sinkt die Druckdifferenz über das Unterstützungssystem 2, insbesondere über die üblicherweise vorgesehene Pumpe (hier nicht dargestellt) des Unterstützungssystems 2, sodass entsprechend das Unterstützungssystem 2 während der Systole ebenfalls einen gestiegenen Fluid-Volumenstrom 7 fördert.

Die hier vorgeschlagene Lösung fußt insbesondere darauf, die zeitliche Änderung des Ventrikelvolumens über eine Impedanzmessung zu erfassen. Dies erlaubt in vorteilhafter Weise, dass bei bekanntem bzw. erfasstem zeitlich aufgelöstem Fluid-Volumenstrom 7 bzw. Pumpenvolumenstrom ($Q_p$) durch Differenzbildung der anderweitig kaum zu messende Aortenklappen- bzw. Bypass-Volumenstrom 24 quantifiziert werden kann.

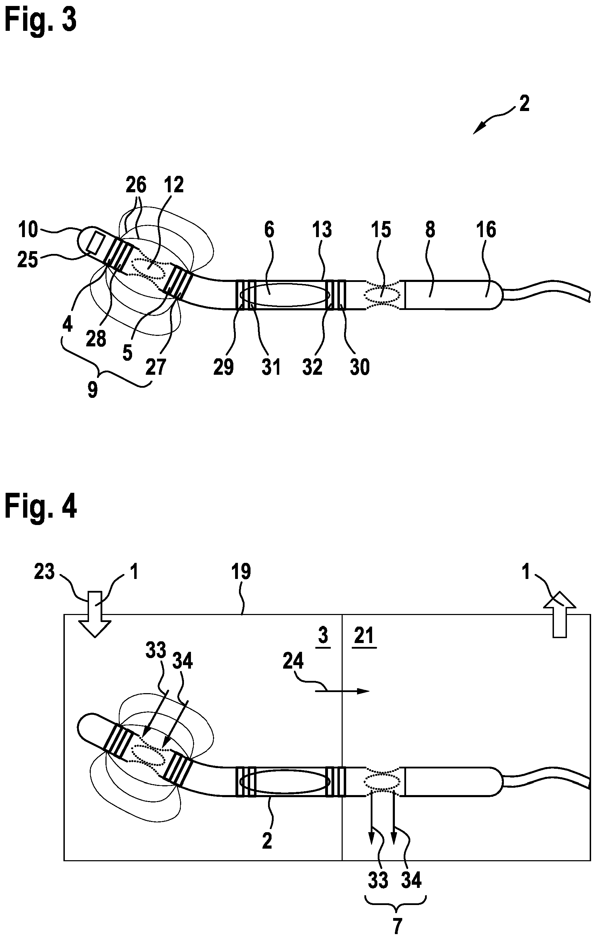

Fig. 3 zeigt schematisch das Unterstützungssystem 2 aus Fig 2. Zum Bestimmen des Ventrikelvolumens zu einem bestimmten Zeitpunkt ist in den Figuren 2 und 3 eine Realisierungsform über eine (linksventrikuläre) intrakardiale Impedanzmessung gezeigt. Die Impedanzmessung hat den Vorteil, dass das gesamte Fluid- bzw. Flüssigkeitsvolumen erfasst und eine elektrische Widerstandsmessung technisch verhältnismäßig einfach umgesetzt werden kann. Durch die hier beispielhafte Verwendung von vier Elektroden 4, 5, 27 und 28 (Vierleitermessung) wird die Impedanzmessung weitgehend unabhängig vom Übergangswiderstand der Messelektroden, sodass das Messergebnis auch bei langfristiger Implantation im Patienten tauglich ist. Die Elektroden 4, 5, 27 und 28 bilden hier eine beispielhafte Messeinrichtung 9 des Unterstützungssystems 2. In Fig. 3 ist zudem beispielhaft veranschaulicht, dass in der Spitze 10 ein ultraschallbasierter Pumpenvolumenstromsensor 25 integriert sein kann. Der Pumpenvolumenstromsensor 25 kann den Fluid-Volumenstrom 7 bzw. den Pumpenvolumenstrom ($Q_p$) erfassen und diesen zeitlich aufgelöst bereitstellen.

Für die Impedanzmessung sollten mindestens zwei Elektroden 4, 27 im Bereich des Ventrikels 20 integriert sein, sodass die Strompfade 26 des Messstroms möglichst gut das Ventrikelvolumen erfassen können. Dazu bietet sich der Bereich des Zulaufkäfigs 11 an, beispielsweise proximal bzw. vor und distal bzw. hinter der bzw. den Zulauföffnungen 12. Technisch sind mindestens zwei Elektroden 4, 27 erforderlich, um den Stromkreis zu schließen. Vorteilhaft ist die Verwendung von vier Elektroden 4, 5, 27 und 28, da so der Einfluss der Kontaktimpedanz von Elektrode zu Blut vernachlässigt werden kann und somit langfristige Veränderungen der Elektrodenoberfläche keinen oder nur einen vernachlässigbaren Einfluss auf das Messergebnis haben. Bevorzugt werden beispielsweise die Stromelektroden 4, 27 außen und die Spannungsmessungselektroden 5, 28 dazwischen platziert. Die Strompfade 26 erstrecken sich dabei von der Elektrode 4 zur Elektrode 27 (Stromelektroden). Dazwischen bilden sich hier nicht gezeigte Äquipotentiallinien aus, die über die Spannungselektroden 28 und 5 hochohmig gemessen werden können.

Da das Erfassungsvolumen insbesondere vom Abstand der Messpaare (4 und 28 zu 5 und 27) abhängig ist, ist es vorteilhaft, ein Pärchen 4, 28 möglichst distal bzw. im Bereich der Spitze 10 und ein Pärchen 5, 27 möglichst proximal bzw. hin zum Elektromotor 16 des Unterstützungssystems 2 zu platzieren. Dabei ist es für die Messqualität vorteilhaft, wenn trotzdem alle vier Elektroden 4, 5, 27 und 28 im Bereich des Ventrikels 20 platziert sind und das proximale Pärchen 5, 27 nicht über die Aortenklappenebene hinaus in die Aorta 21 wandert, wobei auch in diesem Aufbau eine Messung denkbar bzw. realisierbar wäre.

Die gemessene elektrische Leitfähigkeit zwischen den Elektrodenpärchen ist vom umgebenden Fluid- bzw. Flüssigkeitsvolumen und dessen Leitfähigkeit abhängig. Es kann eine vorbestimmte und/oder konstante Leitfähigkeit angenommen werden, da die Ionenkonzentration im Blut durch die Nieren in einem engen Rahmen gehalten wird. Dennoch ist die explizite Bestimmung der Blutleitfähigkeit hier besonders bevorzugt. Dazu ist ein definiertes Volumen 6 erforderlich, wie es beispielsweise im Inneren der (Zulauf-)Kanüle 13 vorliegt. Entsprechend können weitere ein bis vier Elektroden 29, 30, 31 und 32 im Inneren der Kanüle 13 (die auch als Zulaufschlauch bezeichnet werden kann) platziert werden. Dabei kann analog zur Messung im Ventrikelvolumen die Messung durch Stromeinprägung und Spannungsmessung im Elektrodenpaar 29 und 30 erfolgen oder durch Stromeinprägung zwischen dem Elektrodenpaar 29 und 30, sowie Spannungsmessung zwischen dem Elektrodenpaar 31 und 32. Befindet sich mindestens eine Elektrode 4, 5, 27 oder 28 der Ventrikelmessung im Bereich bzw. nahe der Zulauföffnung 12, kann das Pärchen 29, 31 entfallen. Die Messung kann dann beispielsweise durch Stromeinprägung im Elektrodenpaar 27, 30 erfolgen und die Spannungsmessung zwischen dem Elektrodenpaar 5 und 32. Da sich das Elektroden-Pärchen 5, 27 im Bereich bzw. nahe der Zulauföffnung 12 befindet, ergibt sich zwischen dem Elektroden-Pärchen 5, 27 und dem Elektroden-Pärchen 30, 32 ein definiertes Volumen 6 im Innern der Kanüle 13. Im Fall reiner Zweipunktmessungen sind somit insbesondere minimal drei Elektroden notwendig. Die Ventrikelimpedanzmessung erfolgt dann beispielhaft zwischen den Elektroden 4 und 5, die Leitfähigkeitsmessung zwischen den Elektroden 5 und 30.

Die hier dargestellte Zuordnung der Elektroden als Stromelektrode oder Spannungsmessungselektrode ist lediglich beispielhaft. Unter einer Stromelektrode ist hier insbesondere eine an eine Stromquelle anschließbare bzw. angeschlossene Elektrode zu verstehen. Unter einer Spannungsmessungselektrode ist hier insbesondere eine an ein Spannungsmessgerät anschließbare bzw. angeschlossene Elektrode zu verstehen. Es ist jede Zuordnung möglich, die den hier dargestellten Zweck der Impedanzmessung (des Ventrikelvolumens und/oder der Leitfähigkeit) erfüllen kann. Die Spannungsquelle und/oder das Spannungsmessgerät können Bestandteil der Messeinrichtung 9 sein. Befinden sich Stromquelle und Spannungsmessgerät durch eine elektrische Zuleitung 18 räumlich getrennt des Unterstützungssystems 2, ist das Zuleitungskabel besonders vorteilhaft triaxial mit aktiver Schirmung ausgeführt.

Zur Messung (des Ventrikelvolumens und/oder der Leitfähigkeit) kann (Wechsel-)Strom, beispielsweise über eine Stromelektrode eingeprägt und der resultierende Spannungsabfall, beispielsweise an einer Spannungsmessungselektrode gemessen werden. Es kann aber auch Spannung angelegt und der resultierende Stromfluss gemessen werden.

Die Messung kann bei einer einzelnen (Wechselstrom-)Frequenz erfolgen. In der Bioimpedanzanalyse und Impedanz-Tomografie haben sich Werte im Bereich von 50 kHz etabliert. Neben dem Blutvolumen trägt auch die umgebende Herzmuskulatur in geringem Maße zur gemessenen Impedanz bei. Da es sich dabei um zelluläres Material mit anderer Struktur als zelluläres Material in Blut handelt, kann der Einfluss durch Einsatz der sog. Bioimpedanzspektroskopie reduziert werden. Dabei findet die Messung nicht bei einer festen Frequenz, sondern bei mehreren Frequenzen statt. Das Ergebnis ist die Abhängigkeit der elektrischen Impedanz von der Frequenz (vgl. Dispersion, Cole-Diagramm).

Die Messung kann beispielsweise im Bereich von 1 kHz bis 1 MHz erfolgen. Die spektroskopische Messung kann beispielsweise durch eine Abfolge von Sinus-Frequenzen, eine sog. Zirpe (sog. Chirp) oder eine breitbandige Binärfolge (Pseudozufallsrauschen, sog. pseudo random noise) erfolgen.

Die Abtastrate der Ventrikelimpedanz sollte hoch genug sein, um das Nyquist-Theorem für zu erwartende Ventrikelkontraktionsfrequenzen zu erfüllen, beispielsweise 60 1/s. Im Gegensatz zur Volumenänderung des Ventrikels sind Impedanzänderungen der Herzmuskulatur nur sehr langsam zu erwarten, sodass ein vollständiger Frequenzbereichsdurchlauf (sog. Frequenz-Sweep) nicht bei jeder Messung erforderlich ist. Die Bestimmung der Hintergrundimpedanz des Herzmuskels kann periodisch erfolgen. Es ist auch vorteilhaft die Frequenzmesspunkte der Hintergrundimpedanz über mehrere Herzschläge hinweg zu ermitteln. Dazu kann mit hoher Abtastrate die Messung bei zwei Frequenzen erfolgen. Die erste Frequenz ist vorzugsweise fest, die zweite Frequenz wechselt beispielsweise von Herzschlag zu Herzschlag (z. B. ermittelt auf Basis der Impedanzverlaufskurve der ersten Frequenz). Das Spektrum der separaten Messungen wird beispielhaft über die Impedanzverlaufskurve der ersten Frequenz kombiniert. Statt der ersten Frequenz ist auch die Synchronisierung beispielsweise auf die Druckverlaufskurve eines Drucksensors möglich.

Fig. 4 zeigt schematisch ein implantiertes, ventrikuläres Unterstützungssystem 2. In Fig. 4 sind die resultierenden Volumenströme im Herzen 19 verdeutlicht. Das linke Volumen stellt den Ventrikel bzw. das Ventrikelvolumen 3 dar, das rechte Volumen die Aorta 21. Aufgrund von Masseerhaltung entspricht der (Herzklappen-)Volumenstrom 23 vom linken Vorhof in den Ventrikel und der Abfluss in der Aorta dem Fluid-Gesamtvolumenstrom 1 bzw. dem (Gesamt-)HZV. Ausnahmen stellen Klappeninsuffizienz oder hydraulische Kurzschlüsse zwischen den Herzkammern dar.

Das Unterstützungssystem 2 (LVAD) fördert den Förder-Volumenstrom 33, beispielsweise durch die Leistung einer Pumpe (hier nicht dargestellt) des Unterstützungssystems 2. Bei kontinuierlichen Unterstützungssystemen ist dieser Fluss konstant, bei pulsatilen Unterstützungssystemen ist dieser Fluss zeitlich moduliert. Durch die Kontraktion des Ventrikels steigt der Druck im Ventrikel und die Druckdifferenz über das Unterstützungssystem 2 sinkt, sodass das Unterstützungssystem 2 bei konstanter mechanischer Leistung während der Systole einen zusätzlichen Systolen-Volumenstrom 34 fördert. Ist die Ventrikelkontraktion stark genug (beispielsweise bei genesenden Kurzzeitunterstützungspatienten), kann der Ventrikeldruck den Aortenblutdruck überschreiten, was zur Öffnung der Aortenklappe (hier nicht dargestellt) führt. Eine zusätzliche Bypass-Strömung (Formelzeichen $Q_a$) entsteht, die in Fig. 4 als Aortenklappen- bzw. Bypass-Volumenstrom 24 eingetragen ist. Dieser Strömungsanteil (Bypass 24) ist nicht (direkt) durch die Flusssensorik des Unterstützungssystems 2, etwa den Ultraschallsensor in der Spitze des Unterstützungssystems 2 erfassbar, entspricht aber im Wesentlichen der Differenz zwischen Ventrikelvolumenänderung $\partial V/\partial t$ und Pumpenvolumenstrom $Q_p$. Hierbei ergibt sich der Pumpenvolumenstrom $Q_p$, der nur den Fluss durch das Unterstützungssystem 2 selbst quantifiziert und hier auch als Fluid-Volumenstrom 7 bezeichnet wird, aus der Summe von Förder-Volumenstrom 33 und Systolen-Volumenstrom 34. Damit kann durch die Kombination von zeitlicher Ventrikelvolumenmessung und zeitlicher Pumpenflussmessung die Bypassströmung, d. h. der Aortenklappen- bzw. Bypassvolumenstrom 24 (Formelzeichen $Q_A$) nach folgender Gleichung ermittelt werden:

$$Q_A(t) = -\frac{\partial V}{\partial t} + Q_{P,Diastole} - Q_P(t)$$

Wobei hier der Basis-Pumpenvolumenstrom $Q_{P,Diastole}$ ($Q_P$ während der Diastole) dem Förder-Volumenstrom 33 und der zeitabhängige Pumpenvolumenstrom $Q_P(t)$ der Summe von Förder-Volumenstrom 33 und Systolen-Volumenstrom 34 entspricht. $Q_A(t)$ entspricht dem Aortenklappen- bzw. Bypassvolumenstrom 24.

Eine beispielhafte Herleitung dieses Zusammenhangs sowie des Herz-Zeit-Volumens wird anhand der nachstehenden Gleichungen 1 bis 13 veranschaulicht. Die verwendeten Formelzeichen werden dem voranstehend kurz erläutert.

$Q_{HZV}$  Herz-Zeit-Volumen
$Q_P$  Zeitabhängiger Volumenfluss durch die Pumpe
$Q_A$  Zeitabhängiger Volumenfluss durch die Aortenklappe
$Q_V$  Zeitabhängiger Volumenfluss in das Speichervolumen des Ventrikels (Ventrikelvolumenänderung)
$Q_D$  Zeitabhängiger Volumenfluss aus dem linken Vorhof in die Aorta (Annahme: Grundfluss durch die Pumpe)
$V$  Ventrikelvolumen
$t$  Zeit

$$Q_{HZV} = Q_A + Q_P \tag{1}$$

$$Q_{HZV} = Q_D + Q_V \tag{2}$$

$$Q_V = -\frac{\partial V}{\partial t} \tag{3}$$

$$Q_A + Q_P \;=\; Q_D - \frac{\partial V}{\partial t} \tag{4}$$

Annahme: $Q_D$ fließt ausschließlich durch die Pumpe (nicht-pulsatiler Gleichanteil der Strömung). Der pulsatile Anteil der Strömung teilt sich während der Systole auf ein gesteigertes $Q_P$ und eine Bypassströmung durch die Aortenklappe $Q_A$ auf:

$$Q_P \;=\; Q_D + k \cdot \frac{\partial V}{\partial t} \tag{5}$$

$$Q_A \;=\; (1-k) \cdot \frac{\partial V}{\partial t} \tag{6}$$

$$0 < \; k \; \leq 1 \tag{7}$$

Aus der (Bio-)Impedanzmessung ist bekannt:

$$Q_V(t) = \frac{\partial V}{\partial t} \tag{8}$$

Aus einer beispielhaften Ultraschallmessung ist bekannt:

$$Q_P(t) = Q_D + k(t) \cdot \frac{\partial V}{\partial t} \tag{9}$$

Darüber hinaus kann davon ausgegangen werden, dass während der Diastole gilt:

$$Q_{P,Diastole} = Q_D \tag{10}$$

Damit kann die Bypassströmung $Q_A$ ermittelt werden durch:

$$Q_A(t) = Q_V(t) + Q_{P,Diastole} - Q_P(t) \tag{11}$$

und

$$Q_A(t) = \frac{\partial V}{\partial t} + Q_{P,Diastole} - Q_P(t) \tag{12}$$

Zudem kann das Herz-Zeit-Volumen $Q_{HZV}$ ermittelt werden durch:

$$Q_{HZV}(t) = Q_{P,Diastole} - \frac{\partial V}{\partial t} \tag{13}$$

Darüber hinaus ist auch bevorzugt, das Unterstützungssystem in einen speziellen Volumenmessmodus zu versetzen. Dabei wird die Pumpe über integrierte Drucksensoren so geregelt, dass der Druck am Zulaufkäfig und Laufradkäfig identisch ist, also kein Volumenstrom fließt und die Pumpe nur so schnell dreht, dass es nicht zu einem Rückfluss aus der Aorta durch die Pumpe in den Ventrikel kommt. Gleichzeitig kann das Schlagvolumen, Enddiastolische Blutvolumen und/oder die Kontraktionsstärke des Ventrikels basierend auf dem zeitlichen Verlauf der (dabei erfassten) Impedanzmessdaten ermittelt und/oder zur Regelung des Unterstützungssystems verwendet werden.

Neben dem Volumenstrom ist auch das Ventrikelvolumen 3 ein interessanter Parameter. Bei vollunterstützten Systemen (nur die Pumpe fördert Blut) kann es zu einer Saugwirkung (sog. Suction) oder einem Kollabieren des Ventrikels kommen. Dabei fördert das Unterstützungssystem 2 mehr Blut, als aus dem linken Vorhof nachströmt. Das Unterstützungssystem 2 (bzw. dessen Pumpe) leert den Ventrikel. In Folge nähert sich die Ventrikelwand dem Zulaufkäfig und das Unterstützungssystem 2 (bzw. dessen Pumpe) kann sich festsaugen. Bei Unterschreiten eines Ventrikelblutdrucks im Bereich unterhalb 50 mmHg kann es auch zum Kollabieren des Ventrikels durch höhere Umgebungsdrücke kommen. Hier kann das Ventrikelvolumen 3 als Regelparameter genutzt werden, um die Leistung des Unterstützungssystems 2, insbesondere dessen Förder- bzw. Pumpleistung zu reduzieren, sodass ein Mindestventrikelvolumen garantiert werden kann.

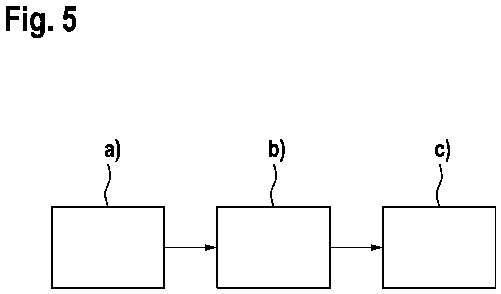

Fig. 5 zeigt das beschriebene Verfahren nochmal in schematischer Form. Die Verfahrensschritte a) Bestimmen eines ersten Impedanzparameters zu einem ersten Zeitpunkt mittels des Unterstützungssystems 2, b) Bestimmen eines zweiten Impedanzparameters zu einem zweiten Zeitpunkt mittels des Unterstützungssystems 2 und c) Zumindest Ermitteln einer Änderung des Impedanzparameters unter Verwendung des ersten Impedanzparameters und des zweiten Impedanzparameters oder Vergleichen zumindest des ersten oder zweiten Impedanzparameters mit einem Schwellenwert werden nacheinander ausgeführt.

Die hier vorgeschlagene Lösung ermöglicht insbesondere einen oder mehrere der nachstehenden Vorteile:

- Nutzung von rein im Unterstützungssystem integrierter Sensorik, es ist beispielsweise keine separate Ultraschallmanschette um die Aorta erforderlich.
- Das Verfahren ermöglicht die Bestimmung von gesamtem Herz-Zeit-Volumen, sowie die Bestimmung des Unterstützungsgrads (Anteil des Pumpenvolumenstroms am gesamten HZV).
- Integration von elektrischer Impedanzmesstechnik ist im Vergleich zu Aorten-Ultraschallmanschetten o.ä. einfach zu realisieren.
- Messung des Gesamt-HZV erfolgt kontinuierlich durch das System und ermöglicht eine Regelung des LVAD sowohl auf das HZV als auch auf den Unterstützungsgrad, was vor allem bei Kurzzeitunterstützungssystemen vorteilhaft ist (Stichwort weaning).
- Das Verfahren bestimmt das Ventrikelvolumen, sodass ein Unterstützungssystem beispielsweise bei Unterschreiten eines minimalen Ventrikelvolumens gedrosselt werden kann.

**Patentansprüche**

1. Implantierbares, ventrikuläres Unterstützungssystem (2), umfassend:

   - eine Messeinrichtung (9), eingerichtet zum Bestimmen eines ersten Impedanzparameters zu einem ersten Zeitpunkt und eines zweiten Impedanzparameters zu einem zweiten Zeitpunkt,
   - eine Verarbeitungseinheit (8), eingerichtet zumindest zum Ermitteln einer Änderung des Impedanzparameters unter Verwendung des ersten Impedanzparameters und des zweiten Impedanzparameters oder zum Vergleichen zumindest des ersten oder zweiten Impedanzparameters mit einem Schwellenwert,
   - wobei die Messeinrichtung (9) mindestens zwei Elektroden (4, 5) umfasst, über die eine Impedanz gemessen werden kann,

   **dadurch gekennzeichnet, dass** die Elektroden zur Ventrikelimpedanzmessung auf oder in der Außenoberfläche des Unterstützungssystems umlaufend angeordnet sind.

2. Unterstützungssystem nach Anspruch 1, wobei die Messeinrichtung (9) vier Elektroden (4, 5, 27, 28) umfasst, über die eine Impedanz gemessen werden kann.

3. Unterstützungssystem nach Anspruch 1 oder 2, wobei die Messeinrichtung (9) zur Impedanzmessung bei unterschiedlichen Frequenzen ausgebildet ist.

4. Unterstützungssystem nach einem der Ansprüche 1 bis 3, weiter umfassend eine Kanüle (13) zur Zuführung eines Fluids und eine Strömungsmaschine (14,16) zur Erzeugung einer Fluidströmung.

5. Unterstützungssystem nach Anspruch 4, wobei die Elektroden auf oder in der Außenoberfläche der Zulauf-Kanüle (13) des Unterstützungssystems umlaufend angeordnet sind.

6. Unterstützungssystem nach einem der Ansprüche 1 bis 5, wobei die Elektroden (4, 5, 27, 28) im implantierten Zustand im Bereich eines Ventrikels (20) eines Herzens platziert sind.

7. Unterstützungssystem nach einem der Ansprüche 1 bis 6, weiter umfassend eine Einrichtung zur Ermittlung eines Fluid-Volumenstroms (7).

8. Verarbeitungseinheit (8), eingerichtet zur Durchführung eines Verfahrens zum Betreiben eines implantierten, ventrikulären Unterstützungssystems (2), wobei das Verfahren folgende Schritte umfasst:

   a) Bestimmen eines ersten Impedanzparameters zu einem ersten Zeitpunkt mittels des Unterstützungssystems (2),
   b) Bestimmen eines zweiten Impedanzparameters zu einem zweiten Zeitpunkt mittels des Unterstützungssystems (2),
   c) Zumindest Ermitteln einer Änderung des Impedanzparameters unter Verwendung des ersten Impedanzparameters und des zweiten Impedanzparameters oder Vergleichen zumindest des ersten oder zweiten Impedanzparameters mit einem Schwellenwert;
   d) wobei die Impedanzparameter über mindestens zwei Elektroden (4, 5) gemessen werden, die an dem Unterstützungssystem (2) angeordnet sind.

9. Verarbeitungseinheit (8) nach Anspruch 8, wobei als weiterer Schritt eine Impedanzmessung bei unterschiedlichen Frequenzen durchgeführt wird.

10. Verarbeitungseinheit (8) nach Anspruch 8 oder 9, wobei als weiterer Schritt die Leitfähigkeit eines Fluids durch eine Impedanzmessung in einem definierten Volumen (6) des Unterstützungssystems (2) ermittelt wird.

11. Verarbeitungseinheit (8) nach einem der Ansprüche 8 bis 10, wobei als weiterer Schritt ein Fluid-Volumenstrom (7) ermittelt wird, der durch das Unterstützungssystem (2) strömt.

12. Verarbeitungseinheit (8) nach einem der Ansprüche 8 bis 11, wobei als weiterer Schritt ein Fluid-Gesamtvolumenstrom (1) im Bereich des Unterstützungssystems (2) ermittelt wird.

**Claims**

1. Implantable ventricular assist system (2) comprising:

   - a measuring device (9), configured to determine a first impedance parameter at a first point in time and a second impedance parameter at a second point in time,
   - a processing unit (8), configured at least to determine a change in the impedance parameter using the first impedance parameter and the second impedance parameter or to compare at least the first or second impedance parameter with a threshold value,
   - wherein the measuring device (9) comprises at least two electrodes (4, 5) via which an impedance can be measured,

   **characterized in that** the electrodes for ventricular impedance measurement are arranged circumferentially on or in the outer surface of the assist system.

2. Assist system according to claim 1, wherein the measuring device (9) comprises four electrodes (4, 5, 27, 28) via which an impedance can be measured.

3. Assist system according to claim 1 or 2, wherein the measuring device (9) is configured for impedance measurement at different frequencies.

4. Assist system according to any of claims 1 to 3, further comprising a cannula (13) for supplying a fluid, and a flow machine (14, 16) for generating a fluid flow.

5. Assist system according to claim 4, wherein the electrodes are arranged circumferentially on or in the outer surface of the inlet cannula (13) of the assist system.

**6.** Assist system according to any of claims 1 to 5, wherein, in the implanted state, the electrodes (4, 5, 27, 28) are placed in the region of a ventricle (20) of a heart.

**7.** Assist system according to any of claims 1 to 6, further comprising a device for determining a fluid volume flow rate (7).

**8.** Processing unit (8) configured to carry out a method for operating an implanted ventricular assist system (2), wherein the method comprises the following steps:

a) determining a first impedance parameter at a first point in time by means of the assist system (2),
b) determining a second impedance parameter at a second point in time by means of the assist system (2),
c) at least determining a change in the impedance parameter using the first impedance parameter and the second impedance parameter or comparing at least the first or second impedance parameter with a threshold value;
d) wherein the impedance parameters are measured via at least two electrodes (4, 5) arranged on the assist system (2).

**9.** Processing unit (8) according to claim 8, wherein, as a further step, an impedance measurement is performed at different frequencies.

**10.** Processing unit (8) according to claim 8 or 9, wherein, as a further step, the conductivity of a fluid is determined by an impedance measurement in a defined volume (6) of the assist system (2).

**11.** Processing unit (8) according to any of claims 8 to 10, wherein, as a further step, a fluid volume flow rate (7) that flows through the assist system (2) is determined.

**12.** Processing unit (8) according to any of claims 8 to 11, wherein, as a further step, a total fluid volume flow rate (1) in the region of the assist system (2) is determined.

**Revendications**

**1.** Système d'assistance (2) ventriculaire implantable, comprenant :

- un dispositif de mesure (9) configuré pour déterminer un premier paramètre d'impédance à un premier moment et un second paramètre d'impédance à un second moment,
- une unité de traitement (8) configurée pour détecter une modification du paramètre d'impédance à l'aide du premier paramètre d'impédance et du second paramètre d'impédance ou pour comparer au moins le premier ou le second paramètre d'impédance à une valeur de seuil,
- dans lequel le dispositif de mesure (9) comprend au moins deux électrodes (4, 5) par l'intermédiaire desquelles une impédance peut être mesurée,

**caractérisé en ce que** les électrodes sont disposées de manière circonférentielle pour la mesure d'impédance ventriculaire sur la surface extérieure du système d'assistance ou dans ladite surface extérieure.

**2.** Système d'assistance selon la revendication 1, dans lequel le dispositif de mesure (9) comprend quatre électrodes (4, 5, 27, 28) par l'intermédiaire desquelles une impédance peut être mesurée.

**3.** Système d'assistance selon la revendication 1 ou 2, dans lequel le dispositif de mesure (9) est conçu pour la mesure d'impédance à des fréquences différentes.

**4.** Système d'assistance selon l'une des revendications 1 à 3, comprenant en outre une canule (13) destinée à l'amenée d'un fluide, et une turbomachine (14, 16) destinée à créer un écoulement de fluide.

**5.** Système d'assistance selon la revendication 4, dans lequel les électrodes sont disposées de manière circonférentielle sur la surface extérieure de la canule d'arrivée (13) du système d'assistance ou dans ladite surface extérieure.

**6.** Système d'assistance selon l'une des revendications 1 à 5, dans lequel, à l'état implanté, les électrodes (4, 5, 27, 28) sont placées dans la zone d'un ventricule (20) du coeur.

**7.** Système d'assistance selon l'une des revendications 1 à 6, comprenant en outre un appareil de détection d'un débit volumique de fluide (7).

**8.** Unité de traitement (8) configurée pour mettre en oeuvre un procédé permettant de faire fonctionner un système d'assistance (2) ventriculaire implanté, dans laquelle le procédé comprend les étapes suivantes :

a) détermination d'un premier paramètre d'impédance à un premier moment à l'aide du système d'assistance (2),
b) détermination d'un second paramètre d'impédance à un second moment à l'aide du système d'assistance (2),
c) au moins détection d'une modification du paramètre d'impédance à l'aide du premier paramètre d'impédance et du second paramètre d'impédance ou comparaison d'au moins le premier ou le second paramètre d'impédance à une valeur de seuil ;
d) dans laquelle les paramètres d'impédance sont mesurés par l'intermédiaire d'au moins deux électrodes (4, 5) qui sont disposées sur le système d'assistance (2).

**9.** Unité de traitement (8) selon la revendication 8, dans laquelle, comme étape supplémentaire, une mesure d'impédance à des fréquences différentes est effectuée.

**10.** Unité de traitement (8) selon la revendication 8 ou 9, dans laquelle, comme étape supplémentaire, la conductivité d'un fluide est détectée par une mesure d'impédance dans un volume (6) défini du système d'assistance (2).

**11.** Unité de traitement (8) selon l'une des revendications 8 à 10, dans laquelle, comme étape supplémentaire, un débit volumique de fluide (7) est détecté, lequel débit volumique circule à travers le système d'assistance (2).

**12.** Unité de traitement (8) selon l'une des revendications 8 à 11, dans laquelle, comme étape supplémentaire, un débit volumique total de fluide (1) est détecté dans la zone du système d'assistance (2).

# Fig. 1

# Fig. 2

EP 3 801 674 B1

## Fig. 3

## Fig. 4

16

# Fig. 5

a) b) c)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2570143 A1
- WO 2016066180 A1
- DE 102009007216 A1